# EUROPEAN PATENT APPLICATION

(11) **EP 1 826 194 A1**
(43) Date of publication of application: **29.08.2007**
(21) Application number: 05819725.2
(22) Date of filing: 04.11.2005
(51) Int. Cl.: C07C 45/39, C07C 49/12

(54) **A PREPARATION METHOD OF BUTADIONE BY GAS-PHASE OXIDATIING 3-HYDROXY-BUTANONE**

(30) Priority: 19.11.2004 CN 200410084382
(71) Applicant: Shanghai Apple Flavor&Fragrance Co. Ltd., Jiading District Shanghai 201809 (CN)
(72) Inventor: CHEN, Hong, Shanghai 201809 (CN); XU, Ping, Shanghai 201809 (CN); XIAO, Zijun, Shanghai 201809 (CN); DU, Yi, Shanghai 201809 (CN); ZENG, Yiyong, Shanghai 201809 (CN); ZHANG, Zhaobin, Shanghai 201809 (CN); MA, Qingyi, Shanghai 201809 (CN); WEI, Zhonghao, Shanghai 201809 (CN)
(74) Representative: Gevers, François
(86) International application number: PCT/CN2005/001850
(87) International publication number: WO 2006/053481

(57) **Abstract**

A novel preparation method for butadione by gas-phase oxidation of 3-hydroxy-butanone is established. The fermentation broth or water solution of 3-hydroxy-butanone is heated to vaporize. The resulting vapour is mixed with air under the molar ratio of oxygen to 3-hydroxy-butanone of 0.5 - 2.5 and then introduced continuously into a fixed bed reactor with activated carbon at the space velocity of 500 h⁻¹ ~ 2500 h⁻¹. The reaction temperature is 110°C ~ 190°C. Butadione is collected by the conventional condensation method at the outlet of the fixed bed reactor. The yield of butadione produced by the present method is 82%. The purity can reach above 98% after rectification. The present method has many advantages such as lower cost, easier operation and controlling, lower reaction temperature, higher reliability, and no substantial pollution.

## Description

### FIELD OF THE INVENTION

The present invention relates to a preparation method of butadione. It especially relates to a novel method for producing butadione by gas-phase oxidation of 3-hydroxyl- butanone.

### BACKGROUND OF THE INVENTION

Butadione is a kind of flavor existing in nature. It is used in foods, beverages, candies, feedingstuff, and essence production. Butadione is also an important material used in chemical industry. For example, it can be used to produce some heterocyclic compounds and serves as the hardening agent of gelatin and the photographic cementing agent. In addition, butadione is a good antiseptic which can kill pathogen at the level of 1 g/L.

The common industrial production method of butadione is to convert methyl ethyl ketone and nitrous acid into butanone oxime. The butanone oxime is decomposed to obtain the product butadione upon the addition of diluted sulfuric acid. However, this method generates wastewater which causes serious environmental pollution. Moreover, the production cost is high.

Another preparation method of butadione used in industry is the so-called ethanol method. Appropriate amounts of nitrous acid, ethanol and water are added into a stirring reactor, while the pre-cooled diluted sulfuric acid is placed in an elevated tank and introduced into the stirring reactor under vacuum to form ethyl ester gas. The ester gas is brought into a reactor filled with butanone to produce ethyl oxime. Then the oxime is distilled under vacuum and the substances with low boiling point are removed. The resulting product acetyl ethyl oxime is put into anther reactor with formaldehyde to get the final product butadione. The most intractable problem when using this method is to control the temperature at which acetyl ethyl oxime is formed. Once the temperature is higher than 80°C, acetyl ethyl oxime will decompose, which may cause detonation. Moreover, since the materials are batchwisely added into the reactors, and it is hard to get rid of the impurities using vacuum distillation, the usage rate of the raw materials is very low.

Vinyl acetylene or methyl vinyl ketone can also be used to produce butadione by hydration and oxidation. But this technique has not been industrialized because of the scarcity of the raw materials and the complexity of the processes.

The method of oxidation of butanone with selenium dioxide or reacting on dimethylglyoxime with sodium nitrite can only be applied in laboratories.

Although there have been many studies on butanedione fermentation using sugars, almost none of them are suitable for industrial production of butanedione mainly due to the low product concentration in the fermentation broths. As for the limited amounts of butanedione products from fermentation on the market, they are mostly the byproducts of 2,3-butanediol fermentation and are expensive.

In the Chinese patent "A Process for Butanedione Production" invented by Zhou Boyuan et al., nitrite methyl ester gas, which was from the reaction of methanol with sodium nitrite and sulfuric acid, was mixed with butanone to produce dimethylglyoxime. The substrates were added at eight intervals. Impurities were removed by steaming. Finally formaldehyde was added to form butanedione. There are three reaction steps in this method, and it is complicated to feed substrates repeatedly. Furthermore, this method also causes environmental pollution.

In the Chinese patent "A Novel Method for Butanedione Preparation" invented by Zhu Fugen et al., gasified isobutyl alcohol was passed through the catalyzing layer comprising silver, copper, zinc, aluminium, and their oxides, and then transformed into butanedione because of molecular rearrangement at high temperature and oxidation by air. Due to the sharp reaction and high temperature up to 200°C ~ 500°C, specific reactors were needed in this method. In addition, the yield of butanedione was low, only amounting to 15% ~ 16%.

### SUMMARY OF THE INVENTION

The present invention discribes a preparation method for butadione by gas-phase oxidation of 3-hydroxyl-butanone, which can conquer the disadvantages of other preparation methods for butadione.

The present invention produces butadione using the fermentation broth or water solution of 3-hydroxyl-butanone as material and atmospheric oxygen as oxidant. The fermentation broth of 3-hydroxyl-butanone is heated to vaporize. The obtained vapour is mixed with air under the molar ration of oxygen to 3-hydroxyl-butanone of 0.5 - 2.5 and then introduced continuously into a fixed bed reactor with activated carbon at the space velocity of 500 h⁻¹ ~ 2500 h⁻¹. The reaction temperature is 110°C ~ 190°C. Butadione is collected by the conventional condensation method at the outlet of the fixed bed reactor.

Therein, the activated carbon can be nut shell activated carbon, wood activated carbon, coal activated carbon or a mixture of the three active carbons at an optional ratio.

Therein, the optimized molar ratio of oxygen entering the fixed-bed reactor to 3-hydroxy-butanone is 0.5 - 1.5.

Therein, the optimized air speed of the mixed gas in the fixed-bed reactor is 1000 h⁻¹ ~ 2000 h⁻¹.

Therein, the optimized reaction temperature is 130°C ~ 170°C.

Therein, the reaction pressure in the above-mentioned fixed-bed reactor is at the absolute pressure of 1.01×10⁵ Pa ~ 2.02×10⁵ Pa.

The chemical formula for the reaction in the above-mentioned fixed-bed reactor is:

2CH₃CHOHCOCH₃ + O₂→ 2CH₃COCOCH₃ + 2H₂O

The preparation method of butanedione provided in the present invention has the following advantages:
1. The product yield is higher. Under the optimized control conditions, the butanedione yield reached 82% and side reactions were fewer. After rectification, butanedione product with the purity of 98% can be obtained.
2. The cost is lower. The main cost to produce butanedione by this method is the cost of raw materials for the 3-hydroxy-butanone fermentation broth. The 3-hydroxy-butanone fermentation broth can be obtained by fermentation of glucose or sucrose, which is cheap and easy to get. So the cost is greatly decreased.
3. It is easy to operate and control. Control of the material ratio and the flow by flowmeter together with control of the reaction temperature by thermocouple are the only requirements.
4. Continuous operation is used. Materials continuously go into the fixed-bed reactor from one end, and the product continuously flows out from the other end. This simplifies some fussy steps in the intermittent operation.
5. The reaction temperature is lower. The optimized reaction temperature is only 130°C ~ 170°C. Therefore, it is convenient to design and manufacture the fixed-bed reactor as well as to select the heating media.
6. It is safe and reliable. The reaction temperature of this preparation method for butanedione is lower, and the reaction pressure in the fixed-bed reactor is only at the absolute pressure of 1.01 × 10⁵ Pa - 2.02 ×10⁵ Pa. It requires no complex controls.
7. It is clean and eco-friendly. There are very few residual materials and byproducts after the reaction, and most of them can be cooled in the flow-up condenser, resulting in no air pollution. The reaction vector activated carbon can be used continuously for a long period and finally be burnt as fuels. The main wastewater is generated in the rectification of butanedione, and its COD is low.

### EXAMPLES

### EXAMPLE 1

### Step 1: preparation of 3-hydroxy-butanone fermentation broth

Preparation of the cell broth of *Bacillus pumilus* XH195 DSM 16187. A loop of the strain *Bacillus pumilus* XH195 DSM 16187 cultured on LBS (LB medium containing high Sugar) slant was inoculated in a 300-ml conical flask containing 50 ml sterilized LBS broth and cultivated at 37°C with shaking at 180 r/min for 24 hours to get the resulting cell broth.

The components of the above-mentioned LBS were as follows. One liter of distilled water contained 200 g of glucose, 10g of peptone, 5.0 g of yeast extract, and 10 g of NaCl. The medium was sterilized at 121°C for 15 min. Twenty grams per liter of agar was added to the above LB broth medium to form the solid medium.

By cultivation of *Bacillus pumilus* XH195 DSM 16187, the mature 3-hydroxy-butanone fermentation broth was obtained from glucose fermentation medium.

The cell broth obtained according to the method above was inoculated at a volume ratio of 50 ml per liter to 300-ml conical flasks each of which contained 50 ml of sterilized glucose fermentation medium. The bacterium was cultured at 37°C with shaking at 180 r/min. Sampling was made every other 4 hours and the concentration of 3-hydroxy-butanone was measured. When the concentration of 3-hydroxy-butanone reached 63.0 g/L at 60 h, the flasks were removed from the shaker to stop fermentation. So the mature 3-hydroxy-butanone fermentation broth was obtained.

The components of the above-mentioned glucose fermentation medium were as follows. One liter of distilled water contained 200 g of glucose, 50 g of NH₄Cl, 0.50 g of KH₂PO₄, 4.0 g of K₂HPO₄·3H₂O, 2.0 ml of 10 g/L of CaCl₂ solution, 2.0 ml of 100 g/L of MgCl₂·6H₂O solution, 200 µl of 10 g/L of FeCl₃ solution, 200 µl of 50 g/L of NaCl solution, 5.0 ml of 10 g/L of yeast extract solution, 5.0 ml of metal ions mixture solution, and 200 µl of vitamin mixture solution. The medium was sterilized at 121°C for 15 min. Therein the components of the metal ions mixture solution were as follows. One liter of distilled water contained 0.50 g of ZnCl₂, 0.50 g of FeCl₃, 0.50 g of MnCl₂·4H₂O, 0.10 g of NaMoO₄·2H₂O, 0.050 g of CuCl₂·2H₂O, 0.050 g of Na₂WO₄·2H₂O, and 120 mmol/L of HC1. Therein the components of the vitamin mixture solution were as follows. One liter of distilled water contained 0.40 g of calcium pantothenate, 0.20 g of inositol, 0.40 g of nicotinic acid, 0.40 g of VB₆, 0.20 g of *p*-aminobenzoic acid, and 0.5 mg of VB₁₂.

*Bacillus pumilus* XH195 mentioned in the above implementation sample was deposited in a microorganism deposit center in Germany (Deutsche Sammlung von Mikroorganismen und Zellkulturen GmbH, Braunschweig) on January 27, 2004. The deposit number is DSM 16187.

The fermentation broth containing 63.0 g/L of 3-hydroxy-butanone was finally obtained by the method above.

Or, 3-hydroxy-butanone fermentation broth could also be prepared from sucrose.

By the use of *Bacillus pumilus* XH195 DSM 16187, the mature 3-hydroxy-butanone fermentation broth was obtained from sucrose fermentation medium. The operation above was repeated with the only change of glucose fermentation medium to sucrose fermentation medium. The concentration of 3-hydroxy-butanone reached 58.1 g/L after 60-hour culture. The above-mentioned sucrose fermentation medium was similar to the glucose fermentation medium with the only change of 200 g of glucose to 180 g of sucrose.

The fermentation broth containing 58.1 g/L of 3-hydroxy-butanone was finally obtained by the method above.

Nut shell activated carbon, wood activated carbon and coal activated carbon were purchased from activated carbon production factories.

### Step 2: preparation of butadione by gas-phase oxidation of 3-hydroxy-butanone using the fermentation broth above

A stainless steel tube, the diameter of which was 38*3 mm and the length of which was 1500 mm, was filled with nut shell activated carbon. The tube was heated by electrothermal wire wrapped outside and the heating temperature was controlled by a booster. The fermentation broth of 3-hydroxy-butanone prepared in step one or 3-hydroxy-butanone solution with the same concentration thereof was heated to vaporize. The obtained vapour was mixed with air under the molar ratio of oxygen to 3-hhydroxy-butanone of 0.5 and then introduced continuously into the stainless steel tube fixed bed reactor at the space velocity of 500 h⁻¹. The reaction temperature was 110°Cₒ The space velocity was controlled by flowmeter while the reaction temperature was controlled by thermocouple. The outlet of the fixed bed reactor was connected to a condenser tube and the water solution of butadione was collected at the other end of the condenser tube. The purity of butadiene reached above 98% after rectification.

The yield of butadione reached 70% in the present example.

### EXAMPLE 2:

Repeat the process of step two in EXAMPLE 1, changing the activated carbon to wood activated carbon, the molar ratio of oxygen to 3-hydroxy-butanone to 0.75, the space velocity of mixed vapour to 1000 h⁻¹, and the reaction temperature to 130°C.

The yield of butadione reached 75%.

### EXAMPLE 3:

Repeat the process of step two in EXAMPLE 1, changing the activated carbon to coal activated carbon, the molar ratio of oxygen to 3-hydroxy-butanone to 1.0, the space velocity of mixed vapour to 1250 h⁻¹, and the reaction temperature to 140°C.

The yield of butadione reached 82%.

### EXAMPLE 4:

Repeat the process of step two in EXAMPLE 1, changing the activated carbon to the mixture of nut shell activated carbon and wood activated carbon (the mass ratio of nut shell activated carbon to wood activated carbon was 1:1), the molar ratio of oxygen to 3-hydroxy-butanone to 1.25, the space velocity of mixed vapour to 1500 h⁻¹, and the reaction temperature to 150°C.

The yield of butadione reached 80%.

### EXAMPLE 5:

Repeat the process of step two in EXAMPLE 1, changing the activated carbon to the mixture of wood activated carbon and coal activated carbon (the mass ratio of wood activated carbon to coal activated carbon was 1:1), the molar ratio of oxygen to 3-hydroxy-butanone to 1:5, the space velocity of mixed vapour to 1750 h⁻¹, and the reaction temperature to 160°C.

The yield of butadione reached 81%.

### EXAMPLE 6:

Repeat the process of step two in EXAMPLE 1, changing the activated carbon to the mixture of nut shell activated carbon and coal activated carbon (the mass ratio of nut shell activated carbon to coal activated carbon was 1:1), the molar ratio of oxygen to 3-hydroxy-butanone to 2.0, the space velocity of mixed vapour to 2000 h⁻¹, and the reaction temperature to 170°C.

The yield of butadione reached 79%.

### EXAMPLE 7:

Repeat the process of step two in EXAMPLE 1, changing the activated carbon to the mixture of nut shell activated carbon, wood activated carbon and coal activated carbon (the mass ratio of nut shell activated carbon, wood activated carbon to coal activated carbon was 1:1:1), the molar ratio of oxygen to 3-hydroxy-butanone to 2.5, the space velocity of mixed vapour to 2500 h⁻¹, and the reaction temperature to 190°C.

The yield of butadione reached 73%.

### EXAMPLE 8:

Repeat the process of step two in EXAMPLE 1 and the process in EXAMPLE 3, replacing the activated carbon with the mixture of nut shell activated carbon, wood activated carbon and coal activated carbon (the mass ratio of nut shell activated carbon, wood activated carbon to coal activated carbon was 3:2:1).

The yield of butadione reached 80%.

### EXAMPLE 9:

Repeat the process of step two in EXAMPLE 1 and the process in EXAMPLE 3, replacing the activated carbon with the mixture of nut shell activated carbon, wood activated carbon and coal activated carbon (the mass ratio of nut shell activated carbon, wood activated carbon to coal activated carbon was1:2:3).

The yield of butadione reached 81 %.

## Claims

1. A novel preparation method for butadione by gas-phase oxidation of 3-hydroxy-butanone. This method has the following characteristics. The fermentation broth or water solution of 3-hydroxy-butanone is heated to vaporize. The obtained vapour is mixed with air under the molar ratio of oxygen to 3-hydroxy-butanone of 0.5 - 2.5 and then introduced continuously into a fixed bed reactor with activated carbon at the space velocity of 500 h⁻¹~ 2500 h⁻¹. The reaction temperature is 110 °C ~ 190°C. Butadione is collected by the conventional condensation method at the outlet of the fixed bed reactor.

2. The process of preparing butadione by gas-phase oxidation of 3-hydroxy-butanone of claim 1, wherein the related activated carbon is one of nut shell activated carbon, wood activated carbon, and coal activated carbon, or a mixture of the three at an optional ratio.

3. The process of preparing butadione by gas-phase oxidation of 3-hydroxy-butanone of claim 1, wherein the mixed gas introduced into the fixed bed reactor is under the molar ratio of oxygen to 3-hydroxy-butanone of 0.5 ~ 1.5.

4. The process of preparing butadione by gas-phase oxidation of 3-hydroxy-butanone of claim 1, wherein the space velocity of the mixed gas in the fixed bed reactor is preferentially 1000 h⁻¹ ~ 2000 h⁻¹ .

5. The process of preparing butadione by gas-phase oxidation of 3-hydroxy-butanone of claim 1, wherein the reaction temperature is preferentially 130°C ~ 170°C.
